(19) Europäisches Patentamt
European Patent Office
Office européen des brevets

(11) **EP 2 985 018 B1**

(12) **FASCICULE DE BREVET EUROPEEN**

(45) Date de publication et mention
de la délivrance du brevet:
**08.01.2020 Bulletin 2020/02**

(51) Int Cl.:
**A61K 8/37** *(2006.01)*    **A61K 8/35** *(2006.01)*
**A61Q 17/04** *(2006.01)*

(21) Numéro de dépôt: **15186109.3**

(22) Date de dépôt: **15.06.2005**

(54) **COMPOSITION ANTI-DÉCOLORATION**

VERFÄRBUNGEN VERHINDERNDE ZUSAMMENSETZUNG

ANTI-DISCOLORATION COMPOSITION

(84) Etats contractants désignés:
**AT BE BG CH CY CZ DE DK EE ES FI FR GB GR HU IE IS IT LI LT LU MC NL PL PT RO SE SI SK TR**

(30) Priorité: **17.06.2004 FR 0406605**

(43) Date de publication de la demande:
**17.02.2016 Bulletin 2016/07**

(62) Numéro(s) de document de la (des) demande(s) initiale(s) en application de l'article 76 CBE:
**05778721.0 / 1 755 543**

(73) Titulaire: **Sensient Cosmetic Technologies**
**95310 Saint Ouen L'Aumone (FR)**

(72) Inventeurs:
• **SEU-SALERNO, Martine**
**95310 SAINT-OUEN-L'AUMONE (FR)**
• **GRIZZO, Serge**
**95310 SAINT-OUEN-L'AUMONE (FR)**

(74) Mandataire: **Lavoix**
**2, place d'Estienne d'Orves**
**75441 Paris Cedex 09 (FR)**

(56) Documents cités:
**EP-A1- 1 454 619    EP-A2- 0 930 063**
**EP-A2- 1 284 130    WO-A1-03/013468**
**WO-A1-03/020224   WO-A2-03/013456**
**WO-A2-03/075877   US-A- 6 136 299**
**US-B1- 6 555 095    US-B2- 6 491 901**

• **LEIBOWITZ M.R. ET AL: "ULTRAVIOLET ABSORBERS AS STABILIZERS IN THE COSMETIC INDUSTRY", JOURNAL OF THE SOCIETY OF COSMETIC CHEMISTS, 1 janvier 1963 (1963-01-01), pages 217-225, XP055148603,**
• **ANONYMOUS: "LCW les colorants Wackherr, COVABSORB", TECHNICAL BULLETIN, 9 octobre 1997 (1997-10-09), page 1, XP003029551,**
• **"Solution in Ethanol (10 mg/l)", LCW CERTIFICAT D'ANALYSES COVABSORB SENSIENT COSMETIC TECHNOLOGIES, 3 mars 2003 (2003-03-03), page 1, XP003029552,**
• **ANONYMOUS: "LCW Certificat d'analyses COVABSORB SENSIENT Cosmetic Technologies 03-03-2003", 20030303, 3 mars 2003 (2003-03-03), page 1, XP003029553,**

- **"CRODA CREATES LIQUID LAUNDRY DETERGENT", HAPPI HOUSEHOLD AND PERSONAL PRODUCTS INDUSTRY, RODMAN PUBLISHING, RAMSEY, NJ, US, 1 janvier 2001 (2001-01-01), pages 1-4, XP003029554, ISSN: 0090-8878 & The Free Library:Household & Personal Products Industry: "CRODA CREATES LIQUID LAUNDRY DETERGENT", , 1 février 2001 (2001-02-01), pages 3-10, ISSN: 0090-8878 Extrait de l'Internet: URL:http://www.thefreelibrary.com/CRODA+CREATES+LIQUID+LAUNDRY+DETERGENT.-a070 391703 [extrait le 2010-02-02] & The Free Library:Household & Personal products Industry: "Reader Service", , 1 février 2001 (2001-02-01), pages 1-7, ISSN: 0090-8878 Extrait de l'Internet: URL:http://www.thefreelibrary.com/_/print/ PrintArticle.aspx?id=71762716 [extrait le 2010-09-13] & "Neue Rohstoffe: Dow Corning introduces SensiGel: The new perfume product category", SOFW JOURNAL, vol. 126, no. 9, 13 novembre 2000 (2000-11-13), page 92,**
- **"CONDEA VISTA DEVOLOPS SPF SUN SPRAY", HAPPI HOUSEHOLD AND PERSONAL PRODUCTS INDUSTRY, RODMAN PUBLISHING, RAMSEY, NJ, US, 1 janvier 2001 (2001-01-01), pages 1-2, XP003029555, ISSN: 0090-8878**
- **Anonymous: "Rouge Covasorb W 3784 A", Sensient Cosmetic Technologies (Technical Data Sheet) , 5 août 2010 (2010-08-05), XP002751206, European Patent Register Extrait de l'Internet: URL:https://register.epo.org/application?d ocumentId=EST8X10W2168FI4&number=EP057 7872 1&lng=en&npl=false [extrait le 2015-11-24]**

Remarques:

Le dossier contient des informations techniques présentées postérieurement au dépôt de la demande et ne figurant pas dans le présent fascicule.

**Description**

La présente invention a trait à des compositions permettant de stabiliser la coloration de formulations, notamment de formulations cosmétiques.

En particulier dans le domaine de la cosmétique, de nombreuses formulations comprennent des colorants, naturels ou synthétiques, qui sont généralement destinés à en améliorer l'aspect et/ou à leur conférer une coloration caractéristique. Une coloration peut également apparaître en raison de la coloration intrinsèque de certains ingrédients, par exemple des parfums.

Dans les deux cas, la coloration de la formulation est due à la présence de composés absorbant la lumière de manière sélective.

Souvent, de telles formulations colorées sont conditionnées dans des emballages transparents à la lumière, par exemple en verre ou en matière plastique, de façon à laisser apparaître les produits. Ces emballages sont généralement également transparents aux rayons ultraviolets (UV).

Or étant des composés qui absorbent la lumière, les colorants sont sensibles à la lumière et plus particulièrement aux rayons ultraviolets particulièrement énergétiques. En effet, l'exposition à des rayons ultraviolets peut induire des réactions radicalaires modifiant la structure chimique des colorants et de ce fait leurs propriétés colorantes. On observe alors une modification de la couleur des formulations, sous forme de décoloration et/ou d'un virement de couleur.

Pour améliorer la stabilité des formulations colorées vis-à-vis des rayons ultraviolets, on peut utiliser des filtres ultraviolets, qui assurent une protection par absorption sélective du rayonnement UV.

A titre de filtres ultraviolets, on a utilisé notamment des composés de la famille des benzophénones.

Ces composés présentent toutefois certains inconvénients. D'une part, ils présentent une coloration jaune prononcée. Cette coloration propre s'ajoute à la coloration initiale, par exemple en transformant une coloration bleue initiale en coloration verte.

De plus, l'efficacité des benzophénones en matière de stabilisation de la coloration s'est révélée insuffisante. En outre, les molécules de type benzophénone sont en général mal adaptées à une utilisation dans des compositions cosmétiques aqueuses. En effet, les benzophénones sont généralement non hydrosolubles, et/ou présentent un caractère acide marqué, ce qui implique une étape supplémentaire d'ajustement du pH de la composition lorsqu'un tel composé est utilisé.

D'autres composés absorbant les rayons ultraviolets sont connus, tels que ceux mentionnés par exemple dans l'annexe VII de la Directive C.E.E. N° 76/168 (relative aux filtres ultraviolets acceptés en cosmétique) ou dans les Directives de la F.D.A. (Food and Drugs Administration américaine), à savoir dans le Federal Register, vol. 43, n° 166 (25 août 1978).

Ces composés sont toutefois destinés à protéger la peau et les cheveux contre les effets nocifs des rayons UV, et non pas les produits cosmétiques eux-mêmes ou leurs constituants, comme les colorants. A ce sujet, on pourra notamment se reporter à l'article "Filtres et écrans solaires" de M.C. Poelman dans «Actifs et additifs en cosmétologie », 2ème édition, 1999, Editions Tec & Doc, Paris.

En effet, des travaux conduits par les inventeurs ont permis d'établir que l'utilisation d'un seul filtre UV dans une formulation n'assure pas toujours une protection efficace de la coloration vis-à-vis des rayons ultraviolets.

Ainsi, la présence d'un filtre UV dans une composition cosmétique ne suffit pas à stabiliser la coloration vis-à-vis des rayons UV, alors qu'il permet de protéger la peau ou les cheveux lorsque la composition y est appliquée.

La présente invention a pour but de fournir une composition permettant d'assurer la stabilisation effective et durable de la coloration contre les effets néfastes des rayonnements ultraviolets et ne présentant pas les inconvénients mentionnés ci-dessus.

Dans ce cadre, l'invention se fixe en particulier pour but de fournir des compositions stabilisantes compatibles avec une utilisation en cosmétique.

A cet effet, selon un premier aspect, la présente invention propose une composition stabilisante consistant en une association de :

(A) 70 à 80% de 2-éthyl-hexyl-méthoxycinnamate (désigné ci-après par "composé A") ; et
(B) 10 à 20% de butyl-méthoxy-dibenzoylméthane (désigné ci-après par "composé B") ; et
(C) 10 à 15% d'éthyl-hexyl-salicylate (désigné ci-après par "composé C"), et éventuellement un tensioactif,
les pourcentages étant en masse par rapport à la masse totale des composés A, B et C,
ladite composition se présentant sous forme liquide.

Cette composition stabilisante est constituée par un mélange des composés (A), (B) et (C), ou bien par un mélange des composés (A), (B) et (C) avec un tensioactif.

Par "filtre ultraviolet" (ou "filtre UV"), on entend, au sens de la présente description, un composé chimique capable d'absorber des rayonnements électromagnétiques dans une gamme comprise dans le domaine des rayonnements ultraviolets, à savoir dans le domaine de longueurs d'ondes comprises entre 4 et 400 nm. L'association des composés A, B et C permet en général une absorption des rayonnements compris entre 200 et 400 mm.

En effet, les inventeurs ont constaté que, de façon surprenante l'association des composés A, B et C assure une stabilisation très efficace des colorants d'une formulation aux rayonnements ultraviolets.

Cette association est d'autant plus intéressante qu'elle est incolore ou très faiblement colorée.

Avantageusement, l'association des composés A, B et C est adaptée à une utilisation dans une formulation cosmétique, chacun des composés A, B et C étant un filtre UV autorisé dans le domaine cosmétique, notamment selon les législations européenne, américaines et japonaises.

Le composé A est un filtre ultraviolet de la famille des cinnamates et est le 2-éthyl-hexyl-méthoxycinnamate.

Le composé B est un filtre UV de la famille des dibenzoylméthanes et est le butyl-méthoxy-dibenzoylméthane (dit aussi "avobenzone").

Enfin, le composé C est un filtre ultraviolet de la famille des salicylates et est l'éthyl-hexyl-salicylate.

La composition stabilisante se présente sous forme liquide, ce qui facilite le dosage et permet une incorporation efficace et homogène dans la formulation.

Toujours en ce qui concerne les teneurs en différents composés A, B et C, les travaux conduits par les inventeurs ont permis d'établir que des résultats de stabilisation de coloration particulièrement intéressants sont observés lorsque le composé A est présent au sein du mélange des composés A, B et C en une teneur d'au moins 70% en masse par rapport à la masse totale des composés A, B et C.

Ainsi, les compositions stabilisantes comprennent de 70 à 80% en masse de composé A par rapport à la masse totale du mélange des composés A, B et C.

Ainsi, la composition stabilisante est une composition dite "Composition 1", comprenant :

- 70 à 80% en masse de composé A, et
- 10 à 20% en masse de composé B, et
- 10 à 15% en masse de composé C,

par rapport à la masse totale du mélange des composés A, B et C.

Ainsi, la Composition 1 peut comprendre :

- 80% en masse de composé A ;
- 10 % de composé B ; et
- 10 % de composé C.

De façon avantageuse, une Composition 2 contient :

70% en masse de composé A ;
15% en masse de composé B ; et
15% de composé C.

Sous sa forme la plus simple, la composition stabilisante est constituée d'un mélange des composés A, B et C.

Les composés A, B et C sont solubles dans la plupart des solvants organiques, notamment dans les alcools et dans les phases lipidiques. De ce fait, la composition stabilisante sous cette forme simple est aisément incorporée dans les compositions cosmétiques comportant des solvants organiques ou des tensioactifs telles que les shampooings, les bains moussants, les eaux de toilette, eaux de parfums et huiles.

La composition stabilisante peut toutefois aussi être solubilisée dans des formulations à base de solvants hydrophiles, et notamment dans des formulations aqueuses en présence d'un tensioactif adapté.

Selon une variante intéressante, la composition stabilisante comporte donc outre les composés A, B et C un tensioactif.

Des tensioactifs particulièrement adaptés sont notamment les tensioactifs non ioniques et en particulier le mélange de tensioactifs non ioniques commercialisé par la société LCW sous le nom de solubilisant LRI.

Le tensioactif est mis en œuvre dans une quantité suffisante pour assurer la solubilisation de la composition stabilisante dans la formulation. Généralement, une quantité de 20 à 40%, et de préférence de l'ordre de 30% en masse par rapport au total des composés A + B + C est appropriée.

Selon cette variante, la composition stabilisante ajoutée à la formulation est solubilisée en formant une émulsion ou micro-émulsion, dans laquelle la phase huileuse comprend les composés A, B et C.

Les compositions stabilisantes décrites permettent de stabiliser la coloration d'une formulation comportant ou non une phase organique. Ainsi, la formulation stabilisée peut être de type solution, émulsion, ou gel et comportant des colorants naturels ou artificiels, hydrosolubles ou liposolubles.

Ainsi, l'utilisation des compositions stabilisantes, à titre d'additif stabilisateur des colorants d'une formulation, constitue un autre objet particulier de la présente invention.

Dans ce cadre, l'invention a en particulier pour objet l'utilisation des compositions stabilisantes précitées pour stabiliser les colorants dans des formulations cosmétiques, telles que des parfums, des eaux de toilette, ou encore des produits de soin ou d'hygiène corporelle tels que des shampooings ou des gels douches, par exemple.

Les utilisations précitées concernent en particulier la stabilisation des colorants sensibles au rayonnement ultraviolet, notamment les colorants organiques tels que le F&DC Red 40, les colorants naturels, comme la garance, le carmin soluble, le caramel, la chlorophylle ou le curcumin.

Afin de préserver la coloration des effets des rayons UV, on ajoute la composition stabilisante à la formulation en une quantité appropriée. Cette quantité dépendra de la nature des colorants, mais aussi du degré d'exposition de la formulation aux rayons UV. Elle sera donc notamment fonction de la transparence du récipient aux rayons UV et de la durée de vie de la formulation.

En principe, une teneur de 0,1 à 3%, de préférence de 0,2% à 2%, et tout particulièrement de 0,5% à 1,5% en masse par rapport à la masse totale de la formulation est suffisante.

Dans le cas particulier de composition aqueuse, il est en général préférable que le mélange des composés A, B et C soit mis en œuvre à raison de 1,5 à 4% en masse par rapport à la masse totale de la formulation stabilisée.

Selon un dernier aspect, la présente invention a pour objet des formulations, notamment cosmétiques, comprenant la composition stabilisante telle que définie précédemment.

La formulation peut comprendre une phase huileuse telle que, par exemple, une huile polaire, une huile apolaire ou un parfum, ou bien encore une phase hydro-alcoolique, comprenant au moins 10%, voire au moins 30% en masse d'un alcool tel que l'éthanol.

Si la formulation comprend une phase aqueuse ou hydro-alcoolique, la composition stabilisante peut être solubilisée grâce à un tensioactif. Le tensioactif peut être déjà présent dans la formulation, ou dans la composition stabilisante ajoutée.

**Mesures colorimétriques**

L'effet stabilisant de la composition est mis en évidence par les mesures colorimétriques suivantes.

On a préparé des solutions des colorants FD&C Blue 1 et FD&C Red 40 dans de l'eau distillée, lesquelles ont été diluées afin d'obtenir une absorbance comprise entre 0,3 et 1. On divise la solution obtenue en deux parties égales, dont une sert de témoins alors que l'autre est exposée à la lumière solaire dans un appareil SUN-TEST de chez Heraeus pendant 72 heures.

Ensuite, on enregistre le spectre d'absorbance de l'échantillon et du témoin de chaque colorant au moyen d'un spectrophotomètre UV-Visible (Varian, Modèle CARY 1) entre 200 et 720 nm. A partir de ces spectres, on détermine les valeurs respectives d'absorbance $A_{max}$ aux maxima d'absorbance pour le témoin et l'échantillon respectivement.

La force colorante de l'échantillon X est alors donnée par la relation suivante :

$$X = \frac{A_{\max(témoin)}}{A_{\max(échantillon)}} x100$$

Un des paramètres permettant de caractériser la coloration d'une formulation est son absorption à une longueur d'onde caractéristique. De préférence, l'absorbance caractéristique dans les formulations comprenant la composition stabilisante après exposition à la lumière pour une durée d'un mois chute de 15% au plus, encore préférée de 10% au plus et tout particulièrement de 8% au plus.

D'autres avantages et caractéristiques de la présente invention apparaîtront de façon plus claire au vu des exemples illustratifs exposés ci-après.

**EXEMPLE 1** : **Préparation d'une composition stabilisante**

On a réalisé une composition stabilisante comprenant 80% en masse d'éthyl-hexyl-méthoxy-cinnamate, 10% en masse de butyl-méthoxy-benzoyl-méthane, et 10% en masse d'éthyl-hexyl-salicylate selon le protocole ci-après.

Dans un mélangeur, on a introduit 80g d'éthyl-hexyl-méthoxy-cinnamate10 g de butyl-méthoxy-dibenzoyl-méthane, et 10 g d'éthyl-hexyl-salicylate.

Le milieu ainsi obtenu a été chauffé à 60° C sous agitation jusqu'à obtention d'un liquide clair.

### EXEMPLE 2 : Préparation d'une composition stabilisante

On a réalisé une composition stabilisante selon le protocole ci-après.

Dans un mélangeur, on a introduit 70g d'éthyl-hexyl-méthoxy-cinnamate, 15g de butyl-méthoxy-dibenzoyl-méthane, et 15g d'éthyl-hexyl-salicylate.

### EXEMPLE 3 comparatif : Préparation d'une composition stabilisante

On a réalisé une composition stabilisante comparative selon le protocole ci-après.

Dans un mélangeur, on a introduit 60g d'éthyl-hexyl-méthoxy-cinnamate, 20g de butyl-méthoxy-dibenzoyl-méthane, et 20g d'éthyl-hexyl-salicylate.

Le milieu ainsi obtenu a été chauffé à 60° C sous agitation jusqu'à obtention d'un liquide clair.

### EXEMPLE 4 comparatif : Préparation d'une composition stabilisante

On a réalisé une composition stabilisante comparative selon le protocole suivant.

Dans un mélangeur on a introduit 90g de butyl-méthoxy-benzoyl-méthane, puis on a introduit 5g d'éthyl-méthoxy-cinnamate et 5g d'éthyl-hexyl-salicylate. On a obtenu, après malaxage d'une durée de 2 minutes un mélange des trois composés se présentant sous la forme d'une poudre.

Les compositions des exemples 1 à 4 sont reportées dans le tableau 1 ci-après, où les pourcentages indiqués sont exprimés par rapport à la masse totale des composés A (éthyl-hexyl-méthoxy-cinnamate), B (butyl-méthoxy-benzoyl-méthane), C (éthyl-hexyl-salicylate).

Tableau 1 : Composition stabilisante des exemples 1 à 4

| | Exemple 1 | Exemple 2 | Exemple 3 | Exemple 4 |
|---|---|---|---|---|
| Composé A Ethyl-hexyl-méthoxycinnamate | 80% | 70% | 60% | 5% |
| Composé B Butyl-méthoxy-benzoyl-méthane | 10% | 15% | 20% | 90% |
| Composé C Ethyl-hexyl-salicylate | 10% | 15% | 20% | 5% |

### EXEMPLE 5 : Formulations comprenant la composition stabilisante

Les compositions stabilisantes des exemples 1 à 4 précédents ont été ajoutées à différentes formulations cosmétiques.

### a- Shampooings.

On a réalisé des formulations de shampooings a1 à a5 selon les indications du tableau 2 ci-dessous, en ajoutant à la fin 1 % en masse par rapport à la formulation finie de composition stabilisante préparée dans les exemples précédents.

Tableau 2 : Formulations de shampooings

| | a1 | a2 | a3 | a4 | a5 (témoin) |
|---|---|---|---|---|---|
| Texapon NSO [sodium lauryléther sulfate] | 30% | 30% | 30% | 30% | 30% |
| Cocoamidopropyl-bétaïne | 10% | 10% | 10% | 10% | 10% |
| Colorant rouge FD&C Red 40 | 0,001% | 0,001% | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |

(suite)

|  | a1 | a2 | a3 | a4 | a5 (témoin) |
|---|---|---|---|---|---|
| Eau | q.s. 100% | q.s. 100% | q.s. 100% | q.s. 100% | q.s. 100% |
| pH | 4,7 | 5,2 | 4,6 | 4,7 | 5,7 |

## b - Compositions huileuses

On a réalisé des compositions huileuses b1 à b5 selon les indications du tableau 3 ci-dessous, en ajoutant à la fin 1 % en masse par rapport à la formulation finie de composition stabilisante préparée dans les exemples précédents.

Tableau 3 : Compositions huileuses

|  | b1 | b2 | b3 | b4 | b5 (témoin) |
|---|---|---|---|---|---|
| Colorant rouge D&C Red 17 | 0,001% | 0,001% | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Huile de Ricin | q.s. 100% | q.s. 100% | q.s. 1 00% | q.s. 1 00% | q.s. 100% |

## c - Eaux de toilette

On a réalisé des compositions eaux de toilette c1 à c5 selon les indications du tableau 4 ci-dessous, en ajoutant au parfum 1% en masse par rapport à la formulation finie de composition stabilisante préparée dans les exemples précédents puis le colorant et l'éthanol.

Tableau 4 : Eaux de toilette

|  | c1 | c2 | c3 | c4 | c5 (témoin) |
|---|---|---|---|---|---|
| Parfum | 5% | 5% | 5% | 5% | 5% |
| Colorant rouge FD&C Red 40 | 0,001% | 0,001 % | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Ethanol 96° | q.s. 100% | q.s. 100% | q.s. 100% | q.s. 100% | q.s. 100% |

## d - Compositions aqueuses

On a réalisé différentes compositions aqueuses d1 à d4 dont les compositions sont reportées dans le tableau 5 ci-après. La composition stabilisante préparée selon les exemples 1 à 4 est mélangée au préalable avec un mélange de tensioactifs non ioniques (solubilisant LRI commercialisé par la société LCW) dans un ratio pondéral de 1:2. Le mélange est ensuite chauffé à 70° C sous agitation jusqu'à obtention d'une solution limpide.

Dans le cas de la composition stabilisante selon l'exemple 4, la solution devient translucide et granuleuse lorsqu'elle est refroidie sous agitation.

Tableau 5 : Compositions aqueuses

|  | d1 | d2 | d3 | d4 | d5 (témoin) |
|---|---|---|---|---|---|
| Colorant bleu FD&C Blue 1 | 0,001% | 0,001% | 0,001% | 0,001% | 0,001% |

(suite)

|  | d1 | d2 | d3 | d4 | d5 (témoin) |
|---|---|---|---|---|---|
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Solubilisant LRI | 2% | 2% | 2% | 2% | - |
| Eau | q.s. 100% | q.s. 100% | q.s. 100% | q.s. 100% | q.s. 100% |
| pH | 4,7 | 5,2 | 4,6 | 4,7 | 5,7 |

Les différentes compositions réalisées ont été exposées à des rayonnements ultraviolets pendant 72 heures dans un appareil SUN TEST. A l'issue de ce traitement, on a mesuré la force colorante comme décrit ci-dessus.

Les résultats sont exposés dans les tableaux 6 à 9 ci-après. Ils ont été évalués comme suit :

chute de 0%-20% de la force de coloration      ++++
chute de 21-40% de la force de coloration      +++
chute de 41-60% de la force de coloration      ++
chute de 61-80% de la force de coloration      +
chute de 81 -100% de la force de coloration     0

Tableau 6 : Shampooings

| Compositions | a1 | a2 | a3 | a4 | a5 (témoin) |
|---|---|---|---|---|---|
| Stabilisation observée | ++++ | ++ | + | ++ | 0 |

Tableau 7 : Compositions huileuses

| Compositions | b1 | b2 | b3 | b4 | b5 (témoin) |
|---|---|---|---|---|---|
| Stabilisation observée | +++ | +++ | +++ | ++ | 0 |

Tableau 8 :Eaux de toilette

| Compositions | c1 | c2 | c3 | c4 | c5 (témoin) |
|---|---|---|---|---|---|
| Stabilisation observée | ++++ | ++++ | +++ | ++++ | 0 |

Tableau 9 : Compositions aqueuses

| Compositions | d1 | d2 | d3 | d4 | d5 (témoin) |
|---|---|---|---|---|---|
| Stabilisation observée | +++ | ++++ | +++ | ++++ | 0 |

Ces résultats montrent clairement un très net effet de stabilisation obtenu dans tous les cas lorsque l'on introduit la composition stabilisante dans les différentes formulations.

## EXEMPLE COMPARATIF

A titre de comparaison, on a réalisé des compositions similaires à celles de l'exemple 5, dans lesquelles on a remplacé les compositions stabilisantes par des composés A, B et C pris isolément. Les compositions ont été testées dans les mêmes conditions que dans l'exemple 5 (exposition aux rayonnements ultraviolets pendant 72 heures).

Les résultats observés sont exposés dans les tableaux 10 à 13 ci-après, d'où il ressort que, dans toutes les compositions testées, la présence du composé A, B ou C seule ne permet pas de stabiliser les colorants. Au contraire, on observe même une dégradation du comportement de la composition sous le rayonnement ultraviolet.

**Tableau 10** : **Compositions de shampooings**

|  | a1' | a2' | A3' | a5' |
|---|---|---|---|---|
| Texapon NSO (sodium lauryl éther sulphate) | 30% | 30% | 30% | 30% |
| Cocoamidopropyl-bétaïne | 10% | 10% | 10% | 10% |
| Colorant rouge : FD&C Red 40 | 0,001% | 0,001% | 0,001% | 0,001% |
| Composé A Ethyl-hexyl-méthoxycinnamate | 1% | | | |
| Composé B Butyl-méthoxy-benzoyl-méthane | | 1% | | |
| Composé C Ethyl-hexyl-salicylate | | | 1% | |
| Eau | q.s. 100% | q.s. 100% | q.s. 100% | q.s. 100% |
| Stabilité vis-à-vis des UV | 0 | 0 | 0 | 0 |

**Tableau 11 : Compositions huileuses**

|  | b1' | b2' | b3' | b5' |
|---|---|---|---|---|
| Colorant rouge : D&C Red 17 | 0,001% | 0,001% | 0,001% | 0,001% |
| Composé A Ethyl-hexyl-méthoxycinnamate | 1% | | | |
| Composé B Butyl-méthoxy-benzoyl-méthane | | 1% | | |
| Composé C Ethyl-hexyl-salicylate | | | 1% | |
| Huile de ricin | q.s. 100% | q.s. 100% | q.s. 100% | q.s. 100% |
| Stabilité vis-à-vis des UV | 0 | 0 | 0 | 0 |

**Tableau 12 : Eaux de toilette**

|  | c1' | c2' | c3' | c5' |
|---|---|---|---|---|
| Parfum | 5% | 5% | 5% | 5% |
| Colorant rouge : FD&C Red 40 | 0,001% | 0,001% | 0,001% | 0,001% |
| Composé A Ethyl-hexyl-méthoxycinnamate | 1% | | | |
| Composé B Butyl-méthoxy-benzoyl-méthane | | 1% | | |
| Composé C Ethyl-hexyl-salicylate | | | 1% | |

(suite)

|  | c1' | c2' | c3' | c5' |
|---|---|---|---|---|
| Ethanol 96° | q.s. 100% | q.s. 100% | q.s. 100% | q.s. 100% |
| Stabilité vis-à-vis des UV | 0 | 0 | 0 | 0 |

**Tableau 13 : Compositions aqueuses**

|  | d1' | d2' | d3' | d5' |
|---|---|---|---|---|
| Colorant bleu : FD&C Blue 1 | 0,001% | 0,001% | 0,001% | 0,001% |
| Composé A Ethyl-hexyl-méthoxycinnamate + solubilisant LRI | 3% |  |  |  |
| Composé B Butyl-méthoxy-benzoyl-méthane + solubilisant LRI |  | 3% |  |  |
| Composé C Ethyl-hexyl-salicylate + solubilisant LRI |  |  | 3% |  |
| Eau distillée | q.s. 100% | q.s. 100% | q.s. 100% | q.s. 100% |
| Stabilité vis-à-vis des UV | ++ | + | ++ | ++ |

**Revendications**

1. Composition consistant en une association de :

   (A) 70 à 80% de 2-éthyl-hexyl-méthoxycinnamate, désigné ci-après par composé A ; et
   (B) 10 à 20% de butyl-méthoxy-dibenzoylméthane, désigné ci-après par composé B ; et
   (C) 10 à 15% d'éthyl-hexyl-salicylate, désigné ci-après par composé C,
   et éventuellement un tensioactif,
   les pourcentages étant en masse par rapport à la masse totale des composés A, B et C,
   ladite composition se présentant sous forme liquide.

2. Composition selon la revendication 1, qui comprend :

   - 80% en masse de composé A ;
   - 10% en masse de composé B ; et
   - 10% en masse de composé C,
   les pourcentages étant en masse par rapport à la masse totale des composés A, B et C.

3. Composition selon la revendication 1, qui comprend :

   - 70% en masse de composé A ;
   - 15% en masse de composé B ; et
   - 15% en masse de composé C,
   les pourcentages étant en masse par rapport à la masse totale des composés A, B et C.

4. Composition selon la revendication 1 **caractérisée en ce qu'**elle comprend un tensioactif.

5. Utilisation d'une composition selon l'une des revendications précédentes, à titre d'additif stabilisateur de la coloration d'une formulation.

6. Utilisation selon la revendication 5, dans laquelle la formulation est une formulation cosmétique.

**7.** Formulation comprenant un colorant et de 0,1 à 4% d'une composition stabilisante selon l'une des revendications 1 à 4.

**8.** Formulation selon la revendication 7, **caractérisée en ce qu'**il s'agit d'une formulation cosmétique.

**Patentansprüche**

**1.** Zusammensetzung bestehend aus einer Kombination von:

(A) 70 bis 80 % 2-Ethylhexylmethoxycinnamat, im Folgenden als Verbindung A bezeichnet; und
(B) 10 bis 20 % Butylmethoxydibenzoylmethan, im Folgenden als Verbindung B bezeichnet; und
(C) 10 bis 15 % Ethylhexylsalicylat, im Folgenden als Verbindung C bezeichnet, und wahlweise einem Tensid, wobei die Prozentsätze Massenprozent sind, bezogen auf das Gesamtgewicht der Verbindungen A, B und C, wobei die Zusammensetzung flüssig ist.

**2.** Zusammensetzung gemäß Anspruch 1, umfassend:

- 80 Gew.-% Verbindung A;
- 10 Gew.-% Verbindung B; und
- 10 Gew.-% Verbindung C,
wobei die Prozentsätze Massenprozent sind, bezogen auf das Gesamtgewicht der Verbindungen A, B und C.

**3.** Zusammensetzung gemäß Anspruch 1, umfassend:

- 70 Gew.-% Verbindung A;
- 15 Gew.-% Verbindung B; und
- 15 Gew.-% Verbindung C,
wobei die Prozentsätze Massenprozent sind, bezogen auf das Gesamtgewicht der Verbindungen A, B und C.

**4.** Zusammensetzung gemäß Anspruch 1, **gekennzeichnet dadurch, dass** sie ein Tensid umfasst.

**5.** Verwendung einer Zusammensetzung gemäß einem der voranstehenden Ansprüche als farbstabilisierendes Additiv einer Formulierung.

**6.** Verwendung gemäß Anspruch 5, wobei die Formulierung eine kosmetische Formulierung ist.

**7.** Formulierung umfassend einen Farbstoff und von 0,1 bis 4 % einer stabilisierenden Zusammensetzung gemäß einem der Ansprüche 1 bis 4.

**8.** Formulierung gemäß Anspruch 7, **gekennzeichnet dadurch, dass** es sich um eine kosmetische Formulierung handelt.

**Claims**

**1.** Composition consisting of an association of:

(A) from 70 to 80% of 2-ethylhexyl methoxycinnamate, denoted compound A hereinbelow; and
(B) from 10 to 20% of butyl methoxydibenzoylmethane, denoted compound B hereinbelow; and
(C) from 10 to 15% of ethylhexyl salicylate, denoted compound C hereinbelow, and optionally a surfactant, the percentages being by mass, based on the total mass of compounds A, B and C, said composition being in liquid form.

**2.** Composition according to claim 1, which comprises:

- 80% by mass of compound A;
- 10% by mass of compound B; and

- 10% by mass of compound C,
the percentages being by mass, based on the total mass of compounds A, B and C.

3. Composition according to claim 1, which comprises:

   - 70% by mass of compound A;
   - 15% by mass of compound B; and
   - 15% by mass of compound C,
   the percentages being by mass, based on the total mass of compounds A, B and C.

4. Composition according to claim 1,
   **characterised in that** it comprises a surfactant.

5. Use of a composition according to any one of the preceding claims as an additive for stabilising the colouring of a formulation.

6. Use according to claim 5, wherein the formulation is a cosmetic formulation.

7. Formulation comprising a colorant and from 0.1 to 4% of a stabilising composition according to any one of claims 1 to 4.

8. Formulation according to claim 7, **characterised in that** it is a cosmetic formulation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Directives de la F.D.A. Food and Drugs Administration américaine, 25 Août 1978, vol. 43

- Filtres et écrans solaires. **M.C. POELMAN.** Actifs et additifs en cosmétologie. 1999